# EUROPEAN PATENT APPLICATION

(11) **EP 4 840 874 A1**
(43) Date of publication of application: **18.11.2026**
(21) Application number: 25738976.7
(22) Date of filing: 08.01.2025
(51) Int. Cl.: C07K 7/06, A61P 21/00, A61P 1/16, A23L 33/18, A61K 38/00

(54) **NOVEL PEPTIDE HAVING ACTIVITIES OF INHIBITING MUSCLE LOSS AND PROMOTING MUSCLE GROWTH, AND USES THEREOF**

(30) Priority: 12.01.2024 KR 20240005392
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); KIM, Seon Soo, Seoul 06188 (KR); JEONG, Ah Reum, Seoul 06188 (KR)
(74) Representative: Heinonen & Co
(86) International application number: PCT/KR2025/000466
(87) International publication number: WO 2025/150903

(57) **Abstract**

The present invention relates to a peptide having the activities of inhibiting sarcopenia, promoting muscle growth, and inhibiting fatty liver. The peptide according to the present invention can promote the proliferation of muscle cells and the differentiation thereof into muscle fibers, has a muscle regeneration effect even under conditions of muscular atrophy, and can significantly inhibit the expression of fat synthesis-related genes/proteins in hepatocytes. Thus, the peptide according to the present invention is expected to be effectively used for inhibiting sarcopenia, promoting muscle growth, and inhibiting fatty liver.

## Description

### [Technical Field]

### [Cross-citation with related applications]

This application claims the benefit of priority based on Korean Patent Application No. 10-2024-0005392 filed on January 12, 2024, and the entire contents of which are incorporated herein as part of this specification.

### [Technical Field]

The present invention relates to a novel peptide having activities of inhibiting muscle loss and promoting muscle growth, and uses thereof. In addition, the present invention relates to the use of the novel peptide for inhibiting fatty liver.

### [Background Art]

With the elderly population rapidly increasing worldwide recently, a report by the United Nations predicts that the population aged 60 or older will exceed 2 billion by 2050. Skeletal muscle is the largest organ, accounting for 50% of total body weight, and muscle loss and muscle weakness are among the major physical changes that occur due to aging. Accordingly, skeletal muscle tends to decrease by about 1% annually in the body after the age of 30, and then decreases rapidly after the age of 65. This rapid decline in muscle mass can have a significant impact on the quality of life, such as by reducing physical ability and increasing the risk of falls and fractures. Reflecting these concerns, the World Health Organization (WHO) officially recognized sarcopenia as a disease in 2017.

Sarcopenia, caused by the degeneration of spinal nerves, motor nerves, or skeletal muscle fibers associated with muscle disease, is one of the representative intractable diseases for which the cause of onset has not yet been identified. According to research conducted so far, it is known that skeletal muscle contraction does not proceed due to the degeneration of motor neurons that induce skeletal muscle contraction, the expression of proteins involved in muscle contraction within the skeletal muscle is reduced, or normal skeletal muscle contraction does not proceed due to the modification of said proteins, and in the long term, said motor neurons or skeletal muscles are deformed into fibrous tissue. Since the fundamental cause of such sarcopenia has not yet been identified and no method has been developed to prevent or restore the degeneration of motor nerves or skeletal muscles, research is currently actively underway to develop a method to slow down the progression of sarcopenia. Currently, exercise, protein, and calorie supplementation are known to be helpful for sarcopenia; however, since the elderly constitute the majority of sarcopenia patients, there is an urgent need for sarcopenia treatments.

Meanwhile, muscle size or muscle mass is regulated by intracellular signaling processes that induce anabolic or catabolic processes occurring within the muscle. Specifically, when signaling responses inducing muscle protein synthesis are dominant over muscle protein degradation, muscle protein synthesis increases, which manifests as hypertrophy or hyperplasia. In addition, muscle cell differentiation and muscle formation are regulated by various muscle regulatory factors. MyoD initiates the expression of muscle-specific genes and induces the differentiation of muscle satellite cells into myoblasts. The induction of myogenin expression by MyoD activity is a critical factor in myoblast fusion and is involved in the formation of myotubes. Muscle fibers formed through this process bundle together to ultimately form muscle.

Against this technological backdrop, various studies are underway to inhibit muscle loss caused by external or intrinsic factors and to promote muscle protein synthesis (Korean Patent No. 10-2064387), but the current situation is still insufficient.

### [Prior Art Document]

### [Patent Documents]

Korean Patent No. 10-2064387

### [Disclosure]

### [Technical Problem]

The inventors have made research efforts to develop an effective active substance that can be used for the prevention, improvement, or treatment of muscle diseases by inhibiting sarcopenia and promoting muscle growth. As a result, the present invention was completed by experimentally confirming that a novel peptide synthesized by the inventors has excellent sarcopenia inhibition and muscle growth promotion activities, as well as an excellent fatty liver inhibition activity.

Accordingly, the objective of the present invention is to provide a novel peptide having various physiological activities such as inhibiting muscle loss, increasing muscle mass, and inhibiting fatty liver, and a pharmaceutical composition or health functional food comprising the same.

### [Technical Solution]

To achieve the objects of the present invention described above,
one aspect of the present invention provides a peptide comprising the amino acid sequence of SEQ ID NO: 1.

Another aspect of the present invention provides a pharmaceutical composition for the prevention or treatment of muscle diseases comprising the peptide as an active ingredient.

Further another aspect of the present invention provides a food composition for the prevention or improvement of muscle diseases comprising the peptide as an active ingredient.

Further another aspect of the present invention provides a pharmaceutical composition for the prevention or treatment of fatty liver comprising the peptide as an active ingredient.

Further another aspect of the present invention provides a food composition for the prevention or improvement of fatty liver comprising the peptide as an active ingredient.

The present invention will be described in detail below.

### 1. Peptide and its activity

According to one aspect of the present invention, a peptide comprising the amino acid sequence of SEQ ID NO: 1 is provided.

### [Amino acid sequence of SEQ ID NO: 1]

### DTVEN

The term "peptide" as used herein refers to a linear molecule formed by amino acid residues being joined together by peptide bonds.

The peptide comprising the amino acid sequence of SEQ ID NO: 1 of the present invention may be used without modification, but variants or fragments of amino acids having different sequences may be used by deletion, insertion or substitution of amino acid residues, or a combination thereof, within a range that does not affect the original activity of the peptide, such as the activity of inhibiting muscle loss, increasing muscle mass, and inhibiting fatty liver.

The peptide of the present invention can be modified by phosphorylation, sulfation, acrylation, glycosylation, methylation, farnesylation, etc., within a range that does not change its activity.

The peptide of the present invention comprises a peptide comprising substantially identical amino acid sequence with the peptide comprising the amino acid sequence of SEQ ID NO: 1, and a variant thereof or an active fragment thereof. The substantially identical amino acid sequence means an amino acid sequence having at least 75%, for example, at least 80%, at least 85%, at least 90%, at least 95%, at least 97%, and at least 98% sequence identity with the amino acid sequence of SEQ ID NO: 1. In addition, the peptide may additionally comprise a targeting sequence, a tag, a labeled residue, or an amino acid sequence prepared for a specific purpose to increase half-life or peptide stability.

The peptide of the present invention may have N-terminal and/or C-terminal modifications induced to select a portion of the amino acid sequence and increase its activity. Through such N-terminal and/or C-terminal modifications, the stability of the peptide of the present invention can be significantly improved, for example, the half-life of the peptide can be increased upon in vivo administration. The term "stability" includes not only stability in vivo that protects the peptide of the present invention from attack by protein-cleaving enzymes in vivo, but also storage stability (e.g., room temperature storage stability).

The above N-terminal modification may be a protecting group selected from the group consisting of an acetyl group, a fluorenylmethoxycarbonyl group, a formyl group, a palmitoyl group, a myristyl group, a stearyl group, and polyethylene glycol (PEG) attached to the N-terminus of the peptide. The above C-terminal modification may be one in which a hydroxyl group (-OH), an amino group (-NH₂), a hydrazino group (-NHNH₂), etc. are attached to the C-terminus of the peptide, but is not limited thereto.

The peptide of the present invention can be manufactured by various methods widely known in the technology to which this invention pertains. For example, the peptide of the present invention can be manufactured according to a chemical synthesis method known in the industry, especially solid-phase synthesis techniques or liquid-phase synthesis techniques.

In addition, the peptide of the present invention is artificially synthesized, or non-naturally occurring or engineered, and the "non-naturally occurring or engineered" means a state created by applying artificial modification, rather than the state in which it exists as it does in nature. Here, the artificial modification may include artificially synthesizing an amino acid sequence by mimicking a plurality of amino acid structures or being manipulated to obtain chemical stability, enhanced pharmacological properties, altered specificity, or reduced antigenicity.

The peptide of the present invention has activity of inhibiting muscle loss and increasing muscle mass.

In addition, the peptide of the present invention has fatty liver inhibitory activity.

Therefore, the peptide of the present invention can be utilized for the prevention, improvement, or treatment of muscle diseases and for the prevention, improvement, or treatment of fatty liver.

In one embodiment, the peptide of the present invention has one or more of the following activities:
(i) activity that promotes the proliferation and differentiation of myoblasts;
(ii) activity that increases the expression levels of pPI3K and p70S6K proteins, which are myogenic markers;
(iii) activity that increases the expression levels of mRNA and proteins of MyoG, Myf4, Myf5, Myf6 and α-actinin, which are muscle fiber differentiation markers;
(iv) activity that increases the expression levels of mRNA and proteins of SIRT1, AKT, mTOR, p70S6K and IGF-1, which are muscle protein synthesis markers;
(v) activity that increases the myotube synthesis capacity of myoblasts; and
(vi) activity that decreases the expression levels of mRNA and proteins of SREBP1 and FAS, which are lipid synthesis markers.

### 2. Composition for the prevention, improvement, or treatment of muscle diseases

### Pharmaceutical composition

According to another aspect of the present invention, a pharmaceutical composition for the prevention or treatment of muscle diseases is provided, comprising as an active ingredient a peptide comprising the amino acid sequence of SEQ ID NO: 1.

The peptide comprising the amino acid sequence of SEQ ID NO: 1 above is the same as the peptide described in item "*1. Peptide and its activity*" above, so the specific description is taken from therein and is not duplicated.

The peptide of the present invention having the activity described above exhibits excellent efficacy in inhibiting muscle loss and increasing muscle mass, and can be used for the prevention or treatment of muscle diseases.

Meanwhile, despite their effective biological activity, conventional functional peptides have shown disadvantages such as not being effectively absorbed into target tissues or cells due to the size of the peptide itself, or being eliminated from the body in a short period of time due to their short half-life. On the other hand, the pharmaceutical composition for the prevention or treatment of muscle diseases of the present invention includes a peptide consisting of about 10 or fewer amino acids as an active ingredient, and accordingly, the cell penetration rate of the active ingredient is excellent, and, for example, when administered topically, an effective preventive or therapeutic effect for muscle diseases can be obtained.

Herein, the term "muscle disease" refers to a muscle disease, condition, or state associated with a decrease in the proliferation or differentiation of myoblasts, and may collectively refer to muscle diseases resulting from, for example, muscle dysfunction, muscle atrophy, or muscle degeneration. The muscle disease may be one or more selected from the group consisting of, for example, muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbar muscular atrophy, myasthenia gravis, myasthenia, muscle degeneration, and cachexia, but is not limited thereto.

In one embodiment, the peptide of the present invention can promote the proliferation and differentiation of myoblasts to inhibit muscle loss and increase muscle mass. Specifically, the peptide of the present invention can promote the proliferation of myoblasts and significantly increase the expression of early differentiation markers, mid-differentiation markers, and late differentiation markers of myoblasts, thereby promoting differentiation into muscle cells or muscle fibers. In addition, the peptide of the present invention can significantly increase the expression of myogenic markers and muscle protein synthesis markers, and can also increase the myoblast's myovascular synthesis ability, and can improve muscle mass recovery/regeneration when myoblasts are damaged by exogenous factors.

Therefore, the peptide of the present invention can be effectively utilized as an active ingredient in a pharmaceutical composition for the prevention or treatment of muscle diseases, and the pharmaceutical composition of the present invention can be effectively utilized for the prevention or treatment of muscle diseases.

Herein, the term "prevention" refers to any act of suppressing or delaying the onset of a disease through the administration of the above composition.

Herein, the term "treatment" means any form of treatment that provides effects to a subject suffering from a disease or at risk of developing a disease, including improvement of the individual's condition (e.g., one or more symptoms), delay of disease progression, delay of symptom onset, or slowing of symptom progression. Accordingly, "treatment" and "prevention" are not intended to mean the cure or complete elimination of symptoms.

Herein, the term "administration" means introducing a specific substance into a subject by any appropriate method, and the pharmaceutical composition of the present invention may be administered through any general route capable of reaching a target in vivo. For example, the route of administration of the pharmaceutical composition of the present invention is not particularly limited, but may be administered orally or parenterally; in the case of parenteral administration, it may be administered by intramuscular injection, intravenous injection, subcutaneous injection, intraperitoneal injection, local administration, transdermal administration, etc., but is not limited thereto.

The dosage of the pharmaceutical composition may be 0.0001 to 1000 µg, 0.001 to 1000 µg, 0.01 to 1000 µg, 0.1 to 1000 µg, or 1.0 to 1000 µg per day, but is not limited thereto, and may be administered in various amounts depending on factors such as the formulation method, method of administration, age, body weight, sex and pathological condition of the patient, food, time of administration, route of administration, excretion rate and response responsiveness. In addition, the number of administrations of the pharmaceutical composition may be 1 to 4 times, 2 to 3 times, or 2 times per day, and the administration period may be 4 weeks or more, 8 weeks or more, 4 weeks to 12 weeks, or 8 weeks to 12 weeks, but is not limited thereto.

The pharmaceutical composition of the present invention may further comprise a therapeutically effective amount of the above-described peptide and/or a suitable carrier, excipient, or diluent commonly used in the manufacture of the pharmaceutical composition.

The term "therapeutic effective amount" means an amount sufficient to achieve the activity or efficacy of the peptide, which is an active ingredient of the pharmaceutical composition of the present invention, and, for example, an amount sufficient to achieve the preventive or therapeutic efficacy of muscle diseases.

Examples of carriers, excipients, or diluents usable in the pharmaceutical composition of the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, or mineral oil.

The pharmaceutical composition of the present invention may be formulated according to conventional methods into oral formulations such as powders, granules, tablets, capsules, suspensions, emulsions, syrups, and aerosols, as well as external preparations, suppositories, and sterile injectable solutions, and may be manufactured in a unit dosage form or placed in a multi-dose container.

When formulating, it is prepared using diluents or excipients such as commonly used fillers, extenders, binders, wetting agents, disintegrants, and surfactants.

Solid dosage forms for oral administration include tablets, pills, powders, granules, capsules, etc., and these solid dosage forms may be prepared by mixing at least one excipient, for example, starch, calcium carbonate, sucrose or lactose, gelatin, etc. with the pharmaceutical composition of the present invention. In addition, in addition to simple excipients, lubricants such as magnesium, styrene, and talc may also be used.

Liquid formulations for oral administration include suspensions, liquid formulations, emulsions, syrups, etc., and in addition to commonly used simple diluents such as water and liquid paraffin, various excipients, such as humectants, sweeteners, flavorings, and preservatives, may be included.

Preparations for parenteral administration include sterile aqueous solutions, non-aqueous solvents, suspensions, emulsions, lyophilized preparations, and suppositories. As non-aqueous solvents and suspensions, propylene glycol, polyethylene glycol, vegetable oils such as olive oil, and injectable esters such as ethyl oleate may be used. As bases for suppositories, Witepsol, Macrogol, Tween 61, cacao oil, laurin oil, glycerogelatin, etc. may be used.

Formulations for transdermal administration may include dusting powders, emulsions, suspensions, oils, sprays, ointments, cream pastes, gels, foams, or solutions. The pharmaceutical composition of the present invention may be an anhydrous ointment and may contain paraffin, particularly low-viscosity paraffin, which is suitable for topical use and is liquid at body temperature, or the natural fat or partially synthetic fat, for example, coconut fatty acid triglycerides, hydrogenated oils, for example, hydrogenated peanut oil or castor oil, fatty acid fractional esters of glycerol, for example, glycerol monostearate and distearate, silicones, for example, polymethylsiloxanes, such as hexamethyldisiloxane or octamethyltrisiloxane, fatty alcohols associated with aqueous creams and increasing water absorption capacity, and sterols, wool waxes, other emulsifiers and/or other additives.

The dosage of the peptide comprising the amino acid sequence of SEQ ID NO: 1 contained in the pharmaceutical composition of the present invention may vary depending on the patient's condition and body weight, the severity of the disease, the form of the drug, the route of administration, and the duration, but may be appropriately selected in some cases. For example, the peptide comprising the amino acid sequence may be administered at a dose of 0.0001 to 1000 mg/kg per day, specifically 0.1 to 1000 mg/kg, and the application may be divided into one or several doses per day. The dosage of the peptide comprising the amino acid sequence of the present invention may be increased or decreased depending on the route of administration, the severity of the disease, gender, body weight, age, etc. Therefore, the dosage does not limit the scope of the present invention in any way.

The pharmaceutical composition of the present invention may include a peptide comprising the amino acid sequence of SEQ ID NO: 1 of the present invention at a concentration of 0.01 µM to 1000 µM, and specifically, the peptide may be included at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited thereto.

The weight ratio between the peptide and the pharmaceutically acceptable carrier may be, for example, 500:1 to 1:500, and as an example, the weight ratio may be 450:1 to 1:450, 400:1 to 1:400, 350:1 to 1:350, 300:1 to 1:300, 250:1 to 1:250, 200:1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

In addition, the peptide may be contained in nanosomes or nanoparticles to further improve, for example, cell permeability or stability issues. For example, the nanosomes may be manufactured using a microfluidizer with lecithin as a raw material, and may be contained within, for example, lecithin particles. Any known method may be used to manufacture the nanosomes. The size of the nanosome particles may be 10 to 200 nm, for example, 10 to 180 nm, 10 to 160 nm, 10 to 140 nm, 10 to 120 nm, 10 to 100 nm, 10 to 80 nm, 10 to 60 nm, 10 to 40 nm, 10 to 20 nm, 50 to 200 nm, 50 to 180 nm, 50 to 160 nm, 50 to 140 nm, 50 to 120 nm, 50 to 100 nm, 50 to 80 nm , or 50 to 60 nm.

Meanwhile, the pharmaceutical composition of the present invention may additionally contain one or more components that improve, alleviate, treat, or prevent muscle diseases, in addition to the peptide comprising the amino acid sequence of SEQ ID NO: 1. The components may be, for example, any one component selected from vitamin C, vitamin B1, vitamin B2, vitamin B6, vitamin H, vitamin PP, pro-vitamin B5, vitamin A, vitamin D, vitamin E, vitamin K1, or carotene, or a mixture thereof, but are not limited thereto.

Another aspect of the present invention provides a method for preventing or treating muscle diseases in a subject in need thereof, the method comprising administering to the subject a peptide comprising the amino acid sequence of SEQ ID NO: 1 or a pharmaceutical composition comprising the peptide as an active ingredient.

In one embodiment, the subject includes a subject requiring prevention or treatment of a muscle disease.

The term "subject" refers to a human or non-human animal, e.g., a human, primate, mammal, or vertebrate.

Additionally, another aspect of the present invention provides a use for the prevention or treatment of a muscle disease of a peptide comprising the amino acid sequence of SEQ ID NO: 1 or a pharmaceutical composition comprising the peptide as an active ingredient.

### Food composition

According to another aspect of the present invention, a food composition for the prevention or improvement of muscle diseases is provided, comprising as an active ingredient a peptide comprising the amino acid sequence of SEQ ID NO: 1.

Among the terms or elements mentioned in the description of the peptide and pharmaceutical composition, those identical to those already mentioned are as described above.

In one embodiment, the peptide of the present invention can promote the proliferation and differentiation of myoblasts to inhibit muscle loss and increase muscle mass. Accordingly, the peptide of the present invention can be effectively utilized as an active ingredient in a food composition for the prevention or improvement of muscle diseases, and the food composition of the present invention can be effectively utilized for the prevention or improvement of muscle diseases.

In this specification, the term "improvement" may mean any action that at least reduces parameters related to the alleviation or treatment of a condition, such as the degree of symptoms.

The food composition may be used by adding the peptide as is or together with other foods or food ingredients, and may be used appropriately according to conventional methods. For example, it may contain various flavoring agents or natural carbohydrates as additional ingredients, as in conventional beverages. Examples of the natural carbohydrates described above may be monosaccharides, e.g., glucose, fructose, etc.; disaccharides, e.g., maltose, sucrose, etc.; and polysaccharides, e.g., conventional sugars such as dextrin, cyclodextrin, etc., and sugar alcohols such as xylitol, sorbitol, erythritol, etc. As flavoring agents other than those described above, natural flavoring agents (thaumatin, stevia extract (e.g., rebaudioside A, glycyrrhizin, etc.)) and synthetic flavoring agents (saccharin, aspartame, etc.) may be advantageously used. The proportion of the natural carbohydrates may be appropriately determined by the choice of a person skilled in the art.

In addition to the above, the food composition may contain various nutritional supplements, vitamins, minerals (electrolytes), flavoring agents such as synthetic and natural flavoring agents, coloring agents and thickening agents (cheese, chocolate, etc.), pectic acid and its salts, alginic acid and its salts, organic acids, protective colloidal thickeners, pH adjusters, stabilizers, preservatives, glycerin, alcohol, carbonating agents used in carbonated beverages, etc. These ingredients may be used independently or in combination, and the proportion of these additives may also be appropriately selected by a person skilled in the art.

In addition, the food composition of the present invention may additionally contain one or more components that improve, alleviate, treat, or prevent muscle diseases, in addition to the peptide comprising the amino acid sequence of SEQ ID NO: 1. The components may be, for example, any one ingredient selected from vitamin C, vitamin B1, vitamin B2, vitamin B6, vitamin H, vitamin PP, pro-vitamin B5, vitamin A, vitamin D, vitamin E, vitamin K1, or carotene, or a mixture thereof, but are not limited thereto.

Meanwhile, the food composition may include a health functional food or dietary composition. Herein, the term "health functional food" refers to a food manufactured and processed in the form of tablets, capsules, powders, granules, liquids, pills, etc., using raw materials or ingredients that have functionality useful to the human body. Herein, "functionality" means obtaining effects useful for health purposes, such as regulating nutrients or physiological actions regarding the structure and function of the human body. The health functional food or dietary food may be manufactured by methods commonly used in the field of the art, and may be manufactured by adding raw materials and ingredients that are commonly added in the field of the art during such manufacturing. In addition, the formulation of the health functional food or dietary food may also be manufactured without restriction as long as it is a formulation recognized as a health functional food or a dietary food. The food composition can be manufactured in various forms of formulations, and unlike general pharmaceuticals, it has the advantages of being made from food as a raw material, thus avoiding side effects that may occur with long-term use of pharmaceuticals, and it is highly portable. Therefore, the health functional food according to one embodiment can be consumed as an adjuvant to enhance the therapeutic effect of muscle diseases.

In addition, there are no limitations on the types of health functional foods to which the food composition according to one embodiment can be used/applied. The food composition comprising the peptide as an active ingredient may be manufactured by mixing other appropriate auxiliary ingredients that may be contained in health functional foods and known additives, depending on the choice of a person skilled in the art. Examples of foods to which it can be added include meats, sausage, bread, chocolate, candies, snacks, confectionery, pizza, ramen, other noodles, chewing gum, dairy products including ice cream, various soups, beverages, tea, drinks, alcoholic beverages, and vitamin complexes, and in addition, it may be manufactured by adding it to juices, teas, jellies, and juices.

### 3. Composition for the prevention or treatment of fatty liver

### Pharmaceutical composition

According to another aspect of the present invention, a pharmaceutical composition for the prevention or treatment of fatty liver is provided, comprising as an active ingredient a peptide comprising the amino acid sequence of SEQ ID NO: 1.

The peptide comprising the amino acid sequence of SEQ ID NO: 1 is the same as the peptide described in item "*1. Peptide and its activity*" above, and among the terms or elements mentioned in the description of the pharmaceutical composition and food composition, those that are the same as those already mentioned in item "2. *Composition for the prevention, improvement, or treatment of muscle diseases*" above are as described above, so the specific description is taken from therein and is not duplicated.

The peptide of the present invention having the activity described above exhibits excellent efficacy in inhibiting fat synthesis and can be used for the prevention or treatment of fatty liver.

In one embodiment, the peptide of the present invention can significantly reduce the expression of factors involved in fat synthesis in liver cells. Therefore, the peptide of the present invention can be effectively utilized as an active ingredient in a pharmaceutical composition for the prevention or treatment of fatty liver, and the pharmaceutical composition of the present invention can be effectively utilized for the prevention or treatment of fatty liver.

Meanwhile, the pharmaceutical composition of the present invention may additionally contain one or more components that improve, alleviate, treat, or prevent fatty liver in addition to the peptide comprising the amino acid sequence of SEQ ID NO: 1.

Another aspect of the present invention provides a method for preventing or treating fatty liver in a subject in need thereof, the method comprising administering to the subject a peptide comprising the amino acid sequence of SEQ ID NO: 1 or a pharmaceutical composition comprising the peptide as an active ingredient.

In one embodiment, the subject includes a subject requiring prevention or treatment of fatty liver.

Additionally, another aspect of the present invention provides a use for the prevention or treatment of fatty liver of a peptide comprising the amino acid sequence of SEQ ID NO: 1 or a pharmaceutical composition comprising the peptide as an active ingredient.

### Food composition

According to another aspect of the present invention, a food composition for preventing or improving fatty liver is provided, comprising as an active ingredient a peptide comprising the amino acid sequence of SEQ ID NO: 1.

Among the terms or elements mentioned in the description of the peptide and pharmaceutical composition, those identical to those already mentioned are as described above.

In one embodiment, the peptide of the present invention can significantly reduce the expression of factors involved in fat synthesis in liver cells. Therefore, the peptide of the present invention can be effectively utilized as an active ingredient in a food composition for the prevention or improvement of fatty liver, and the food composition of the present invention can be effectively utilized for the prevention or improvement of fatty liver.

Meanwhile, the food composition of the present invention may additionally contain one or more ingredients that improve, alleviate, treat, or prevent fatty liver in addition to the peptide comprising the amino acid sequence of SEQ ID NO: 1.

### [Advantageous Effects]

The peptide of the present invention has sarcopenia-inhibiting, muscle growth-promoting, and fatty liver-inhibiting activities. Specifically, the peptide according to the present invention can promote the proliferation of muscle cells and the differentiation thereof into muscle fibers, has a muscle regeneration effect even under conditions of muscular atrophy, and can significantly inhibit the expression of fat synthesis-related genes/proteins in hepatocytes. Thus, the peptide according to the present invention is expected to be effectively used for inhibiting sarcopenia, promoting muscle growth, and inhibiting fatty liver.

However, the effects of the present invention are not limited to those mentioned above, and other effects not mentioned will be clearly understood by those skilled in the art from the following description.

### [Description of Drawings]

FIG. 1 shows that the peptide of the present invention promotes cell proliferation of C2C12 myoblasts.
FIG. 2 shows that the peptide of the present invention increases the protein expression levels of pPI3K and p70S6K, which are muscle-forming related proteins, in C2C12 cells.
FIGS. 3A and 3B show that the peptide of the present invention increases the mRNA expression levels of MyoG, Myf6, Myf5, and Myf4 genes, which are genes related to early/mid-stage differentiation, in C2C12 cells.
FIGS. 4A and 4B show that the peptide of the present invention increases the protein expression levels of Myf5, MyoG, and α-actinin, which are proteins related to early/mid/late-stage differentiation, in C2C12 cells.
FIGS.5A and 5B show that the peptide of the present invention increases the mRNA expression levels of SIRT1, mTOR, AKT, P70S6K, and IGF-1 genes, which are genes related to muscle protein synthesis, in C2C12 cells.
FIGS. 6A and 6B show that the peptide of the present invention increases the protein expression levels of SIRT1, IGF-1, pmTOR, and p70S6K, which are proteins related to muscle protein synthesis, in C2C12 cells.
FIG. 7 shows that the peptide of the present invention increases myotube synthesis capacity in C2C12 cells.
FIG. 8 shows that the peptide of the present invention restores the protein expression levels of MyoD and α-actinin, which are differentiation-related proteins, in C2C12 cells where an atrophy environment is induced.
FIG. 9 shows that the peptide of the present invention inhibits the mRNA expression levels of SREBP1 and FAS genes, which are lipid synthesis-related genes, in HepG2 cells.
FIG. 10 shows that the peptide of the present invention inhibits the protein expression level of SREBP1, which is a lipid synthesis-related protein, in HepG2 cells.

### [Best Mode]

Hereinafter, the present invention will be described in detail by way of examples. However, the following examples are intended to specifically illustrate the present invention, and the scope of the present invention is not limited by the following examples.

### Preparation Example 1: Preparation of peptide

A peptide having the amino acid sequence SEQ ID NO: 1 listed in Table 1 below was synthesized using an automated peptide synthesizer (Milligen 9050, Millipore, USA), and the synthesized peptides were purified using C18 reverse-phase high-performance liquid chromatography (HPLC) (Waters Associates, USA). An ACQUITY UPLC BEH300 C18 column (2.1 mm × 100 mm, 1.7 µm, Waters Co, USA) was used.

**[Table 1]**

| SEQ ID NO | Amino acid sequence (N-terminus → C-terminus) |
|---|---|
| 1 | DTVEN |

The efficacy of the peptide of SEQ ID NO: 1 prepared above was evaluated through the following experiment.

### Test Example 1: Analysis of myoblast proliferation activity of peptide

To analyze the effect of the peptide prepared in Preparation Example 1 on myoblast proliferation, cell viability/cytotoxicity tests were performed.

C2C12 cells (mouse myoblast line) were cultured in DMEM medium containing 1% P/S and 10% FBS. When the cell confluency reached approximately 70 to 80%, cells were seeded into 96-well culture plates at a density of 5 x 10³ cells/well by 200 µl. After 1 day, the medium was replaced with serum-free DMEM medium (1% P/S), and after 4 hours, it was replaced with DMEM containing 0.2% FBS while adding the peptide of SEQ ID NO: 1 at concentrations of 5 µM, 50 µM, or 100 µM. After 48 and 72 hours, the reaction was carried out using EZ-cytox (DoGenBio Co., Ltd, Seoul, Korea) until the absorbance of Non reached 1.0, and the absorbance at 450 nm was measured using a Spectrophotometer (SPECTRAMAX M2e).

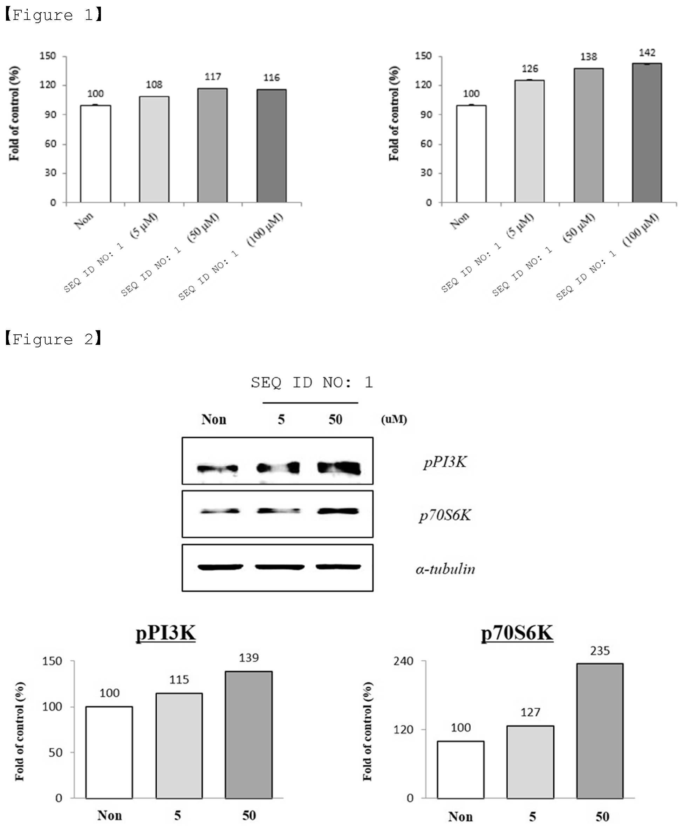

Experimental results showed that the cell proliferation level in C2C12 myoblasts increased in a concentration-dependent manner due to the peptide of SEQ ID NO: 1. Specifically, when treated with the peptide of SEQ ID NO: 1 for 48 hours, it was confirmed that the proliferation level of C2C12 myoblasts increased by approximately 17% at a peptide concentration of 50 µM, and when treated with the peptide of SEQ ID NO: 1 for 72 hours, it was confirmed that the proliferation level of C2C12 myoblasts increased by approximately 42% at a peptide concentration of 100 µM (FIG. 1).

### Test Example 2: Analysis of myogenesis-related protein expression

To determine the effect of the peptide prepared in Preparation Example 1 on myogenesis, the expression levels of myogenesis-related protein markers in myoblasts were evaluated through Western blot analysis.

C2C12 cells (mouse myoblast line) were cultured in DMEM medium containing 1% P/S and 10% FBS. When cell confluency reached approximately 70 to 80%, cells were seeded into 6-well culture plates at a density of 5 x 10⁵ cells/well by 2 ml. After 1 day, the medium was replaced with serum-free DMEM medium (1% P/S), and after 4 hours, the peptide of SEQ ID NO: 1 was added at a concentration of 5 µM or 50 µM. After 1 hour, lysis buffer was added to lyse the cells, which was then centrifuged at 4°C and 12,000 rpm for 30 minutes to obtain the protein, which was quantified using a BCA kit. SDS-PAGE was performed on the protein, and it was electrotransferred onto a membrane. After blocking the protein-electrophoresed membrane with 5% skim milk, anti-pPI3K primary antibody (Cell signaling Technology, USA) and anti-p70S6K primary antibody (Cell signaling Technology, USA) were reacted with anti-β-actin primary antibody (Santa Cruz, USA) as a loading control overnight at 4°C. The membrane was washed with PBS-T, and Peroxidase-conjugated AffiniPure Goat Anti-Rabbit IgG (H+L) secondary antibody (Jackson immunoResearch, USA) and Peroxidase-conjugated AffiniPure Goat Anti-Mouse IgG (H+L) secondary antibody (Jackson immunoResearch, USA) were reacted at room temperature for 1 hour. After washing the membrane again with PBS-T, visualization was performed using Gel Doc (Bio-Rad, Hercules, CA, USA) with Western detection reagent (Elpis Biotech, Daejeon, Korea).

Experimental results showed that the expression of pPI3K and p70S6K, which are myogenesis-related protein markers, increased in a concentration-dependent manner by the peptide of SEQ ID NO: 1 in C2C12 myoblasts. Specifically, it was confirmed that pPI3K increased by approximately 39% at a peptide concentration of 50 µM, and p70S6K increased significantly by approximately 135% at a peptide concentration of 50 µM (FIG. 2).

### Test Example 3: Analysis of gene expression related to early/mid-stage differentiation

To determine the effect of the peptide prepared in Preparation Example 1 on the differentiation of myoblasts into muscle cells/muscle fibers, the expression levels of gene markers related to early/mid-stage differentiation in myoblasts were evaluated through RT-PCR analysis.

C2C12 cells (mouse myoblast line) were cultured in DMEM medium containing 1% P/S and 2% BCS. When cell confluency reached approximately 70 to 80%, cells were seeded into 6-well culture plates at a density of 5 x 10⁵ cells/well by 2 ml. Subsequently, when cell confluency reached 100%, the medium was replaced with differentiation medium (1% P/S, 2% horse serum), and the peptide of SEQ ID NO: 1 was added at a concentration of 5 µM or 50 µM. Afterwards, the differentiation medium and the peptide of SEQ ID NO: 1 were replaced every two days, and three days after the first replacement, the cells were harvested and RNA was extracted using Trizol (Thermo Fisher Scientific, USA). DNA complementary to the extracted RNA was obtained using TOPScript^{™} RT DryMIX (enzynomics, Korea), and then polymerase chain reaction (PCR) was performed on early/mid-stage differentiation marker genes using TOPsimple^{™} DryMIX-nTaq (enzynomics, Korea). The mRNA expression levels of the markers were then compared under each sample treatment condition by running the samples on a 1.5% agarose gel. The primer sequences of the genes used in the experiment and the primer sequence of the GAPDH gene as a control are shown in Table 2.

**[Table 2]**

| Primer | Sequence (5'-> 3') | SEQ ID NO |
|---|---|---|
| MyoG Forward | (5') ACC AGG AGC CCC ACT TCT AT (3') | 2 |
| MyoG Reverse | (5') ACG ATG GAC GTA AGG GAG TG (3') | 3 |
| Myf6 Forward | (5') ATC AGC TAC ATT GAG CGT CTA CA (3') | 4 |
| Myf6 Reverse | (5') CCT GGA ATG ATC CGA AAC ACT TG (3') | 5 |
| Myf5 Forward | (5') TAT GAA GGC TCC TGT CC (3') | 6 |
| Myf5 Reverse | (5') ACG TGC TCC TCA TCG TCT G (3') | 7 |
| Myf4 Forward | (5') ATC AGC TAC ATT GAG CGT CTA CA (3') | 8 |
| Myf4 Reverse | (5') CCT GGA ATG ATC CGA AAC ACT TG (3') | 9 |
| GAPDH Forward | (5') GTG ATG GCA TGG ACT GTG GT (3') | 10 |
| GAPDH Reverse | (5') GGA GCC AAA AGG GTC ATC AT (3') | 11 |

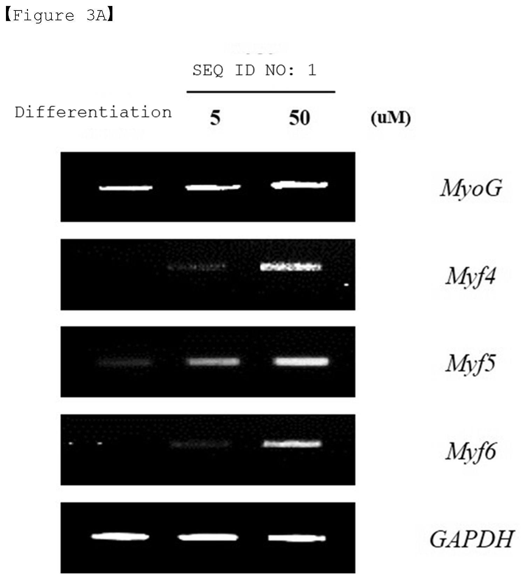

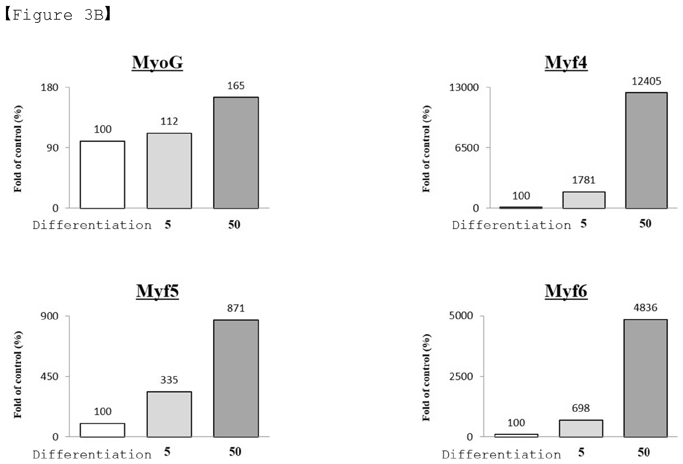

Experimental results confirmed that the mRNA expression levels of MyoG, Myf4, Myf5, and Myf6 genes, which are gene markers related to early/mid-stage differentiation of myoblasts, were significantly increased in a peptide concentration-dependent manner by the peptide of SEQ ID NO: 1 in C2C12 myoblasts (FIG. 3A). In particular, the mRNA expression levels of Myf4 and Myf6 were found to increase significantly by more than 100-fold and more than 48-fold, respectively, at a peptide concentration of 50 µM (FIG. 3B).

### Test Example 4: Analysis of protein expression related to early/mid/late differentiation

To determine the effect of the peptide prepared in Preparation Example 1 on the differentiation of myoblasts into muscle cells/muscle fibers, the expression levels of protein markers related to early/mid/late differentiation in myoblasts were evaluated through Western blot analysis.

C2C12 cells (mouse myoblast line) were cultured in DMEM medium containing 1% P/S and 2% BCS. When cell confluency reached approximately 70 to 80%, cells were seeded into 6-well culture plates at a density of 5 x 10⁵ cells/well by 2 ml. Subsequently, when cell confluency reached 100%, the medium was replaced with differentiation medium (1% P/S, 2% horse serum), and the peptide of SEQ ID NO: 1 was added at a concentration of 5 µM or 50 µM. Subsequently, the differentiation medium and the peptide of SEQ ID NO: 1 were replaced every two days. Three days after the first replacement, lysis buffer was added to lyse the cells, which was then centrifuged at 4°C and 12,000 rpm for 30 minutes to obtain the protein, which was quantified using a BCA kit. SDS-PAGE was performed on the protein, and the protein was electrotransferred onto a membrane. After blocking the protein-electrophoresed membrane with 5% skim milk, the anti-Myf5 primary antibody (Abcam, UK), the anti-MyoG primary antibody (Santa Cruz, USA), and the anti-α-actinin primary antibody (Santa Cruz, USA) were reacted with the anti-β-actin primary antibody (Santa Cruz, USA) as a loading control overnight at 4°C. The membrane was washed with PBS-T, and Peroxidase-conjugated AffiniPure Goat Anti-Rabbit IgG (H+L) secondary antibody (Jackson immunoResearch, USA) and Peroxidase-conjugated AffiniPure Goat Anti-Mouse IgG (H+L) secondary antibody (Jackson immunoResearch, USA) were reacted at room temperature for 1 hour. After washing the membrane again with PBS-T, visualization was performed using Gel Doc (Bio-Rad, Hercules, CA, USA) with Western detection reagent (Elpis Biotech, Daejeon, Korea).

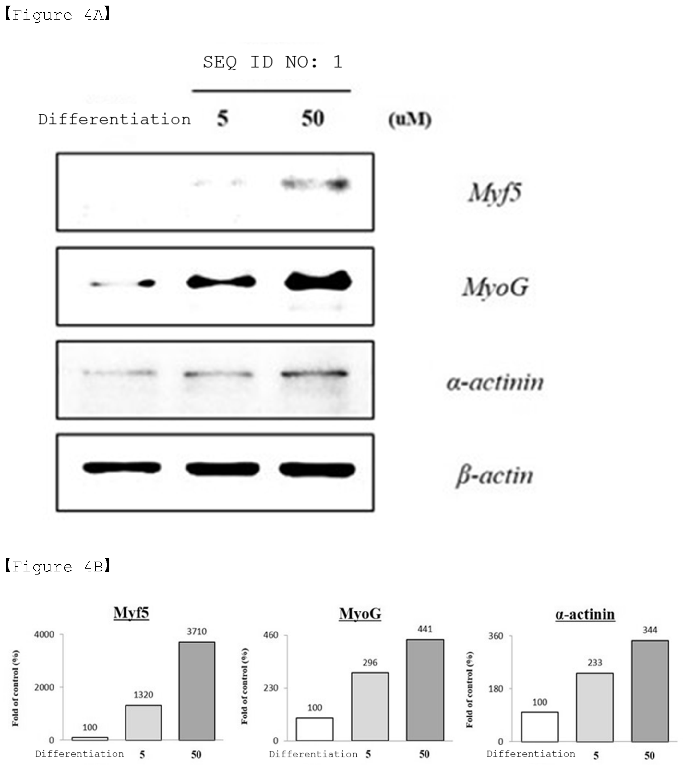

Experimental results confirmed that the expression of Myf5, MyoG, and α-actinin, protein markers related to early, mid, and late differentiation of myoblasts, increased in a concentration-dependent manner by the peptide of SEQ ID NO: 1 in C2C12 myoblasts (FIG. 4A). In particular, in the case of Myf5, the protein expression level was found to increase significantly by more than 37-fold at a peptide concentration of 50 µM (FIG. 4B).

### Test Example 5: Analysis of gene expression related to muscle protein synthesis

To determine the effect of the peptide prepared in Preparation Example 1 on the expression of genes related to muscle protein synthesis, the expression levels of signaling gene markers related to muscle protein synthesis in myoblasts were evaluated through RT-PCR analysis.

C2C12 cells (mouse myoblast line) were cultured in DMEM medium containing 1% P/S and 2% BCS. When cell confluency reached approximately 70 to 80%, cells were seeded into 6-well culture plates at a density of 5 x 10⁵ cells/well with 2 ml aliquots. Subsequently, when cell confluency reached 100%, the medium was replaced with differentiation medium (1% P/S, 2% horse serum), and the peptide of SEQ ID NO: 1 was added at a concentration of 5 µM or 50 µM. Subsequently, the differentiation medium and peptide of SEQ ID NO: 1 were replaced every two days, and cells were harvested three days after the first replacement to extract RNA using Trizol (Thermo Fisher Scientific, USA). DNA complementary to the extracted RNA was obtained using TOPScript^{™} RT DryMIX (enzynomics, Korea), and then polymerase chain reaction (PCR) was performed on signaling genes related to muscle protein synthesis using TOPsimple^{™} DryMIX-nTaq (enzynomics, Korea). The mRNA expression levels of the markers were then compared under each sample treatment condition by running the samples on a 1.5% agarose gel. The primer sequences of the genes used in the experiment and the primer sequence of the GAPDH gene as a control are shown in Table 3.

**[Table 3]**

| Primer | Sequence (5'-> 3') | SEQ ID NO |
|---|---|---|
| SIRT1 Forward | (5') GAT CCT TTG GAT TCC TGC AA (3') | 12 |
| SIRT1 Reverse | (5') AGT TCC AGC CGT CTC TGT GT (3') | 13 |
| mTOR Forward | (5') TTG AGG TCG CTA TGA CCA GAG AGA A (3') | 14 |
| mTOR Reverse | (5') TTA CCA GAA GGG ACA CCA GCC AAT G (3') | 15 |
| AKT Forward | (5') GGT AGC GAT AAT GGA GGT CT (3') | 16 |
| AKT Reverse | (5') GCT TCT GTC CTC TTC TCC TT (3') | 17 |
| P70S6K Forward | (5') GGA GCC TGG GAG CCC TGA TGT (3') | 18 |
| P70S6K Reverse | (5') GAA GCC CTC TTT GAT GCT GTC C (3') | 19 |
| IGF-1 Forward | (5') TGC TCT CAA CAT CTC CCA TC (3') | 20 |
| IGF-1 Reverse | (5') GCC TCC TTA GAT CAC AGC TCC (3') | 21 |
| GAPDH Forward | (5') GTG ATG GCA TGG ACT GTG GT (3') | 10 |
| GAPDH Reverse | (5') GGA GCC AAA AGG GTC ATC AT (3') | 11 |

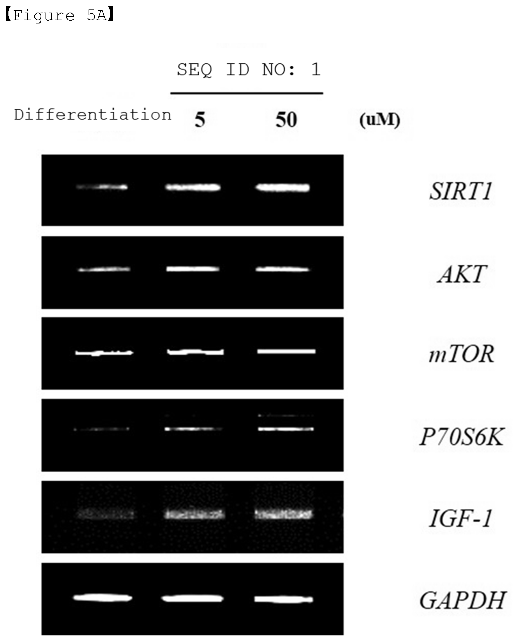

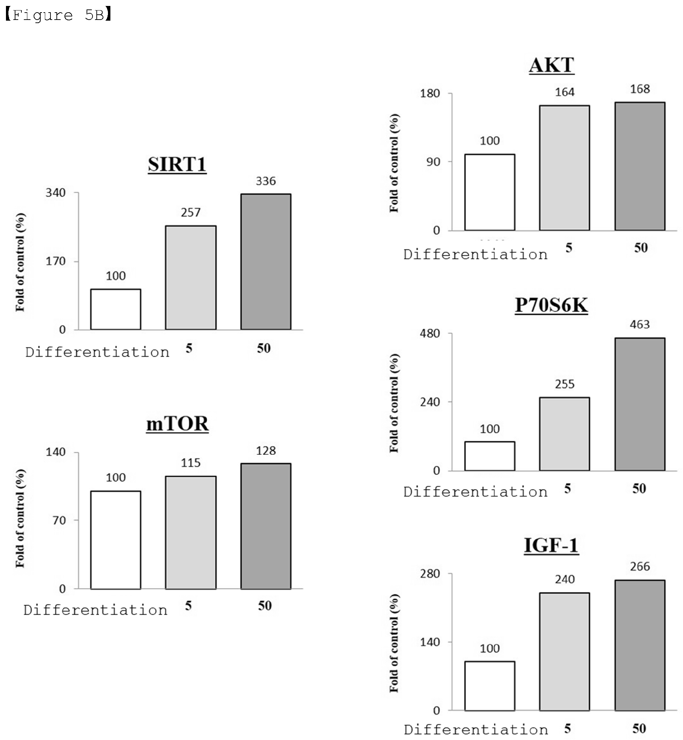

Experimental results confirmed that the mRNA expression levels of SIRT1, AKT, mTOR, P70S6K, and IGF-1 genes, which are muscle protein synthesis-related gene markers, were significantly increased in a peptide concentration-dependent manner by the peptide of SEQ ID NO: 1 in C2C12 myoblasts (FIG. 5A and FIG. 5B).

### Test Example 6: Analysis of protein expression related to muscle protein synthesis

To determine the effect of the peptide prepared in Preparation Example 1 on the expression of proteins related to muscle protein synthesis, the expression levels of signaling protein markers related to muscle protein synthesis in myoblasts were evaluated through Western blot analysis.

C2C12 cells (mouse myoblast line) were cultured in DMEM medium containing 1% P/S and 2% BCS. When cell confluency reached approximately 70 to 80%, cells were seeded into 6-well culture plates at a density of 5 x 10⁵ cells/well with 2 ml aliquots. Subsequently, when cell confluency reached 100%, the medium was replaced with differentiation medium (1% P/S, 2% horse serum), and the peptide of SEQ ID NO: 1 was added at a concentration of 5 µM or 50 µM. Subsequently, the differentiation medium and the peptide of SEQ ID NO: 1 were replaced every two days. Three days after the first replacement, lysis buffer was added to lyse the cells, which was then centrifuged at 4°C and 12,000 rpm for 30 minutes to obtain the protein, which was quantified using a BCA kit. SDS-PAGE was performed on the protein, and the protein was electrotransferred onto a membrane. After blocking the protein-electrophoresed membrane with 5% skim milk, the anti-SIRT1 primary antibody (Cell signaling Technology, USA), anti-IGF-1 primary antibody (Cell signaling Technology, USA), anti-pmTOR primary antibody (Cell signaling Technology, USA), and anti-p70S6K primary antibody (Cell signaling Technology, USA) were reacted with the anti-β-actin primary antibody (Santa Cruz, USA) as a loading control overnight at 4°C. The membrane was washed with PBS-T, and the Peroxidase-conjugated AffiniPure Goat Anti-Rabbit IgG (H+L) secondary antibody (Jackson immunoResearch, USA) and the Peroxidase-conjugated AffiniPure Goat Anti-Mouse IgG (H+L) secondary antibody (Jackson immunoResearch, USA) were incubated for 1 hour at room temperature. After washing the membrane again with PBS-T, visualization was performed using a Gel Doc (Bio-Rad, Hercules, CA, USA) with a Western detection reagent (Elpis Biotech, Daejeon, Korea).

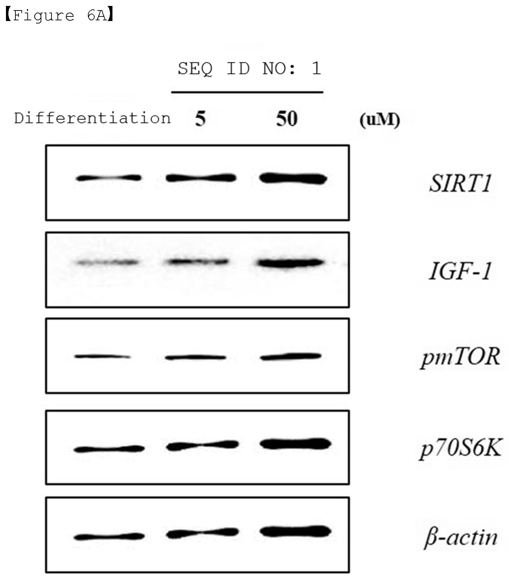

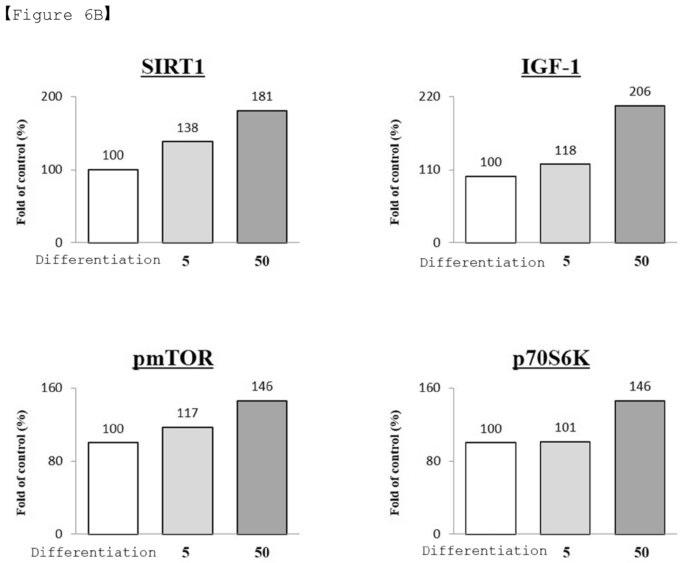

Experimental results confirmed that the protein expression levels of SIRT1, IGF-1, pmTOR, and p70S6K, which are protein markers related to muscle protein synthesis, were significantly increased in a peptide concentration-dependent manner by the peptide of SEQ ID NO: 1 in C2C12 myoblasts (FIG. 6A and FIG. 6B).

### Test Example 7: Analysis of myotube synthesis capacity

To analyze the effect of the peptide prepared in Preparation Example 1 on myotube synthesis capacity in myoblasts, the thickness of the myotubes was compared by observing differentiated myoblasts under a microscope.

C2C12 cells (mouse myoblast line) were cultured in DMEM medium containing 1% P/S and 2% BCS. When cell confluency reached approximately 80%, cells were seeded into 6-well culture plates at a density of 5 x 10⁵ cells/well with 2 ml aliquots. Subsequently, when cell confluency reached 100%, the medium was replaced with differentiation medium (1% P/S, 2% horse serum), and the peptide of SEQ ID NO: 1 was added at a concentration of 5 µM or 50 µM. Afterwards, the differentiation medium and SEQ ID NO: 1 peptide were replaced every two days, and images were taken with an optical microscope (FLEXACAM C1, Leica, Germany) 3 days after differentiation induction to compare the thickness of the myotubes.

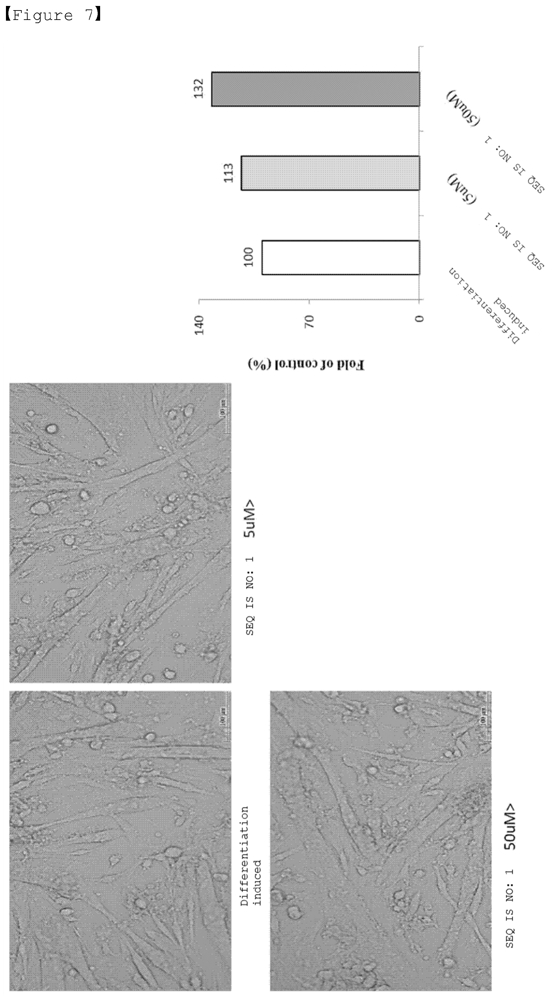

Experimental results showed that myotubes formed by the peptide of SEQ ID NO: 1 in C2C12 myoblasts were fused with surrounding myotubes to form thicker myotubes, and confirmed that this occurred in a peptide concentration-dependent manner (FIG. 7).

### Test Example 8: Analysis of differentiation-related protein expression in muscle atrophy-induced myoblasts

Western blot analysis was used to evaluate whether the peptide prepared in Preparation Example 1 could restore the expression levels of differentiation-related proteins that were reduced in myoblasts with induced muscle atrophy.

C2C12 cells (mouse myoblast line) were cultured in DMEM medium containing 1% P/S and 2% BCS. When cell confluency reached approximately 70 to 80%, cells were seeded into 6-well culture plates at a density of 5 x 10⁵ cells/well by 2 ml. Subsequently, when cell confluency reached 100%, the medium was replaced with differentiation medium (1% P/S, 2% horse serum) to induce differentiation. Subsequently, the differentiation medium was replaced every two days, and on the fourth day of differentiation induction, the cells were pretreated with the peptide of SEQ ID NO: 1 at a concentration of 5 µM or 50 µM for 30 minutes. Next, the cells were treated with soluble dexamethasone (Sigma, USA) and cultured for 24 hours to induce an atrophy environment. After culture, lysis buffer was added to lyse the cells, which was then centrifuged at 4°C and 12,000 rpm for 30 minutes to obtain the protein, which were then quantified using a BCA kit. SDS-PAGE was performed on the protein, and the protein was electrotransferred onto the membrane. After blocking the protein-electrophoresed membrane with 5% skim milk, the anti-MyoD primary antibody (Santa Cruz, USA) and the anti-α-actinin primary antibody (Santa Cruz, USA) were reacted with the anti-α-tubulin primary antibody (Santa Cruz, USA) as a loading control overnight at 4°C. The membrane was washed with PBS-T, and the Peroxidase-conjugated AffiniPure Goat Anti-Rabbit IgG (H+L) secondary antibody (Jackson ImmunoResearch, USA) was reacted with the Peroxidase-conjugated AffiniPure Goat Anti-Mouse IgG (H+L) secondary antibody (Jackson ImmunoResearch, USA) for 1 hour at room temperature. After washing the membrane again with PBS-T, visualization was performed using a Gel Doc (Bio-Rad, Hercules, CA, USA) with a Western detection reagent (Elpis Biotech, Daejeon, Korea).

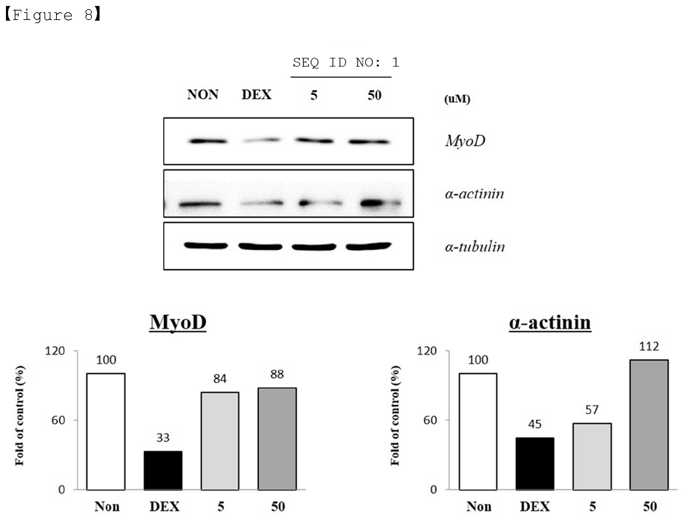

Experimental results showed that when C2C12 myoblasts were treated with dexamethasone, the expression levels of differentiation-related proteins, MyoD and α-actinin were significantly reduced to less than half, but the reduced protein expression levels were restored in a peptide concentration-dependent manner by the peptide of SEQ ID NO: 1 (FIG. 8).

### Test Example 9: Analysis of gene expression related to fat synthesis

To determine the effect of the peptide prepared in Preparation Example 1 on the expression of genes related to lipid synthesis, the expression levels of lipid synthesis-related gene markers in hepatocytes were evaluated through RT-PCR analysis.

HepG2 cells (human hepatocyte line) were cultured in DMEM medium containing 1% P/S and 10% FBS. When cell confluency reached approximately 70 to 80%, cells were seeded into 12-well culture plates at a density of 1 x 10⁵ cells/well by 1 ml. Subsequently, when cell confluency in the 12-well culture plates reached approximately 70 to 80%, the medium was replaced with serum-free DMEM medium. After 4 hours, the medium was replaced with serum-free DMEM (1% BSA) supplemented with 500 µM oleic acid (Sigma, USA), and the cells were treated with the peptide of SEQ ID NO: 1 at concentrations of 5 µM or 50 µM and cultured for 48 hours. After culture, the cells were harvested and RNA was extracted using Trizol (Thermo Fisher Scientific, USA). DNA complementary to the extracted RNA was obtained using TOPScript^{™} RT DryMIX (enzynomics, Korea), and then polymerase chain reaction (PCR) was performed on lipid synthesis-related genes using TOPsimple^{™} DryMIX-nTaq (enzynomics, Korea). The mRNA expression levels of the markers were then compared under each sample treatment condition by running the samples on a 1.5% agarose gel. The primer sequences of the genes used in the experiment and the primer sequence of the GAPDH gene as a control are shown in Table 4.

**[Table 4]**

| Primer | Sequence (5'-> 3') | SEQ ID NO |
|---|---|---|
| SREBP1 Forward | (5') GAC CGA CAT CGA AGG TGA AG (3') | 22 |
| SREBP1 Reverse | (5') AAG AGA GGA GCT CAA TGT GGC (3') | 23 |
| FAS Forward | (5') CGG AAA CTG CAG GAG CTG TC (3') | 24 |
| FAS Reverse | (5') CAC GGA GTT GAG CCG CAT (3') | 25 |
| GAPDH Forward | (5') GAG TCA ACG GAT TTG GTC GT (3') | 26 |
| GAPDH Reverse | (5') GAC AAG CTT CCC GTT CTC AG (3') | 27 |

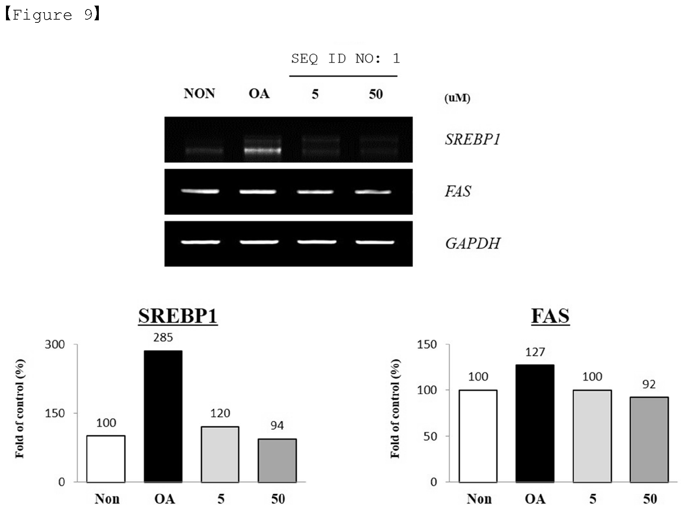

Experimental results showed that when HepG2 hepatocytes were treated with oleic acid, the mRNA expression levels of SREBP1 and FAS genes, which are genes related to lipid synthesis, increased significantly, but the mRNA expression levels of the increased genes decreased in a peptide concentration-dependent manner by the peptide of SEQ ID NO: 1 (FIG. 9).

### Test Example 10: Analysis of fat synthesis-related protein expression

To determine the effect of the peptide prepared in Preparation Example 1 on the expression of proteins related to fat synthesis, the expression levels of fat synthesis-related protein markers in hepatocytes were evaluated through Western blot analysis.

HepG2 cells (human hepatocyte line) were cultured in DMEM medium containing 1% P/S and 10% FBS. When cell confluency reached approximately 70 to 80%, cells were seeded into 12-well culture plates at a density of 1 x 10⁵ cells/well by 1 ml. Subsequently, when cell confluency in the 12-well culture plates reached approximately 70 to 80%, the medium was replaced with serum-free DMEM medium. After 4 hours, the medium was replaced with serum-free DMEM (1% BSA) supplemented with 500 µM oleic acid (Sigma, USA), and the peptide of SEQ ID NO: 1 was treated at a concentration of 5 µM or 50 µM and incubated for 48 hours. After incubation, lysis buffer was added to lyse the cell, which was then centrifuged at 4°C and 12,000 rpm for 30 minutes to obtain the protein, which was quantified using a BCA kit. SDS-PAGE was performed on the protein, and the protein was electrotransferred onto a membrane. After blocking the protein-electrophoresed membrane with 5% skim milk, the anti-SREBP1 primary antibody (Abcam, UK) was reacted with the anti-β-actin primary antibody (Santa Cruz, USA) as a loading control overnight at 4°C. The membrane was washed with PBS-T, and the Peroxidase-conjugated AffiniPure Goat Anti-Mouse IgG (H+L) secondary antibody (Jackson ImmunoResearch, USA) was incubated for 1 hour at room temperature. After washing the membrane again with PBS-T, visualization was performed using a Gel Doc (Bio-Rad, Hercules, CA, USA) with a Western detection reagent (Elpis Biotech, Daejeon, Korea).

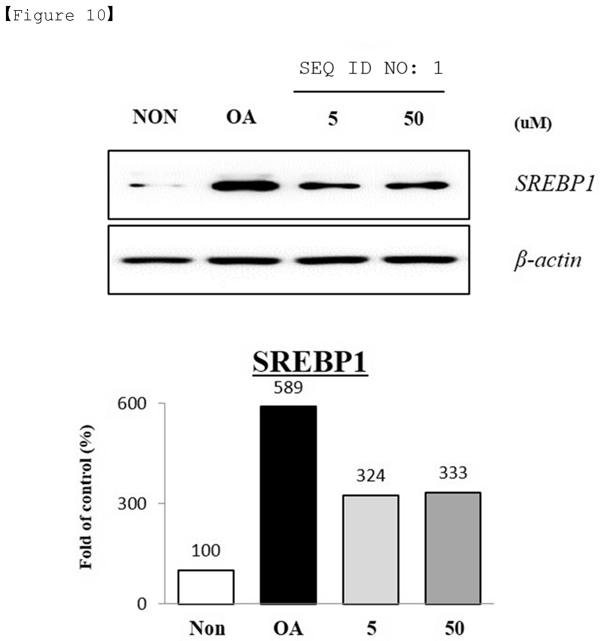

Experimental results showed that when HepG2 hepatocytes were treated with oleic acid, the protein expression level of SREBP1, which is a protein related to lipid synthesis, increased significantly, but the increased protein expression level was reduced by the peptide of SEQ ID NO: 1 (FIG. 10).

Through the above experiments, it was confirmed that the peptide of SEQ ID NO: 1 of the present invention can promote the proliferation of muscle cells and differentiation thereof into muscle fibers, and has a muscle regeneration effect even under conditions of muscular atrophy. In addition, it was confirmed that the peptide of SEQ ID NO: 1 of the present invention can significantly suppress the expression of genes/proteins related to fat synthesis in liver cells. Therefore, the peptide of SEQ ID NO: 1 of the present invention is expected to be effectively utilized for the inhibition of sarcopenia, the promotion of muscle growth, and the inhibition of fatty liver.

Although representative embodiments of the present application have been described above by way of example, the scope of the present application is not limited only to such specific embodiments, and those skilled in the art will be able to make appropriate modifications within the scope described in the claims of the present application.

## Claims

1. A peptide comprising the amino acid sequence of SEQ ID NO: 1.

2. The peptide of claim 1, wherein the peptide has the activities of inhibiting muscle loss and promoting muscle growth.

3. The peptide of claim 1, wherein the peptide has the activity of inhibiting fatty liver.

4. The peptide of claim 1, wherein the peptide has one or more of the following activities:
(i) activity that promotes the proliferation and differentiation of myoblasts;
(ii) activity that increases the expression levels of pPI3K and p70S6K proteins, which are myogenic markers;
(iii) activity that increases the expression levels of mRNA and proteins of MyoG, Myf4, Myf5, Myf6 and α-actinin, which are muscle fiber differentiation markers;
(iv) activity that increases the expression levels of mRNA and proteins of SIRT1, AKT, mTOR, p70S6K and IGF-1, which are muscle protein synthesis markers;
(v) activity that increases the myotube synthesis capacity of myoblasts; and
(vi) activity that decreases the expression levels of mRNA and proteins of SREBP1 and FAS, which are lipid synthesis markers.

5. A pharmaceutical composition for the prevention or treatment of muscle diseases comprising the peptide of claim 1 as an active ingredient.

6. The pharmaceutical composition for the prevention or treatment of muscle diseases of claim 5, wherein the muscle diseases are one or more selected from the group consisting of muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbarmuscular atrophy, myasthenia gravis, myasthenia, muscle degeneration, and cachexia.

7. A food composition for the prevention or improvement of muscle diseases comprising the peptide of claim 1 as an active ingredient.

8. The food composition for the prevention or improvement of muscle diseases of claim 7, wherein the muscle diseases are one or more selected from the group consisting of muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbar muscular atrophy, myasthenia gravis, myasthenia, muscle degeneration, and cachexia.

9. A pharmaceutical composition for the prevention or treatment of fatty liver comprising the peptide of claim 1 as an active ingredient.

10. A food composition for the prevention or improvement of fatty liver comprising the peptide of claim 1 as an active ingredient.

11. A method for preventing or treating muscle diseases in a subject in need thereof, the method comprising administering to the subject the peptide of claim 1 or a pharmaceutical composition comprising the peptide of claim 1 as an active ingredient.

12. The method for preventing or treating muscle diseases of claim 11, wherein the muscle diseases are one or more selected from the group consisting of muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbar muscular atrophy, myasthenia gravis, myasthenia, muscle degeneration, and cachexia.

13. A method for preventing or treating fatty liver in a subject in need thereof, the method comprising administering to the subject the peptide of claim 1 or a pharmaceutical composition comprising the peptide of claim 1 as an active ingredient.
